# EUROPEAN PATENT APPLICATION

(11) **EP 4 468 238 A1**
(43) Date of publication of application: **27.11.2024**
(21) Application number: 23305828.8
(22) Date of filing: 25.05.2023
(51) Int. Cl.: G06T 7/00

(54) **STENOSIS LOCALISATION**

(71) Applicant: Koninklijke Philips N.V., 5656 AG Eindhoven (NL)
(72) Inventor: WIEMKER, Rafael, 5656 EINDHOVEN (NL); BONTUS, Claas, 5656 EINDHOVEN (NL); NICKISCH, Hannes, 5656 EINDHOVEN (NL); MATUTE FLORES, Jose Alejandro, 5656 EINDHOVEN (NL); VLACHOMITROU, Anna Sesilia, 5656 EINDHOVEN (NL); PETERS, Jochen, 5656 EINDHOVEN (NL); HEESE, Harald, 5656 EINDHOVEN (NL); NEMPONT, Olivier, 5656 EINDHOVEN (NL)
(74) Representative: Philips Intellectual Property & Standards

(57) **Abstract**

A computer-implemented method of identifying a potential location of a stenosis in a vessel, is provided. The method includes: estimating, from medical image data, an actual perfusion map representing a spatial distribution of perfused blood in adjoining tissue (140). The medical image data is also analyzed in order to generate, for each of a plurality of candidate locations (160_{1..j}) along the vessel(s), an expected perfusion map (170_{1..j}) representing a spatial distribution of perfused blood in the adjoining tissue (140) as a result of blood flowing downstream of the candidate location. The method also includes: calculating, for each of a plurality of the candidate locations (160_{1..j}), i) a correlation value representing a correlation between the expected perfusion map (170_{1..j}), and the actual perfusion map, or ii) a perfused blood value representing an amount of perfused blood in the actual perfusion map within an expected perfused blood boundary (200_{i..j}) defined by a spatial extent of the perfused blood in the expected perfusion map (170_{1..j}). One or more of the candidate locations (160_{1..j}) are identified as the potential location of the stenosis based on the correlation values, or the perfused blood values, respectively.

## Description

### TECHNICAL FIELD

The present disclosure relates to identifying a location of a stenosis in a blood vessel. A computer-implemented method, a computer program product, and a system, are disclosed.

### BACKGROUND

A vascular stenosis is a term used to describe a narrowing in a blood vessel lumen. Vascular stenoses can occur in arteries, and in veins, and have the effect of limiting blood flow in the vessel. Vascular stenoses can occur in various parts of the body, and have various associated causes. For instance, atherosclerosis is a condition in which plaque builds up in the arteries, reducing the diameter of the arterial lumen. This reduces the supply of blood to tissues in the body. Atherosclerosis has various names, depending on the arteries that are affected. For instance, obstructive coronary artery disease, "CAD" refers to the build-up of plaque in the arteries of the heart, and peripheral artery disease, "PAD", refers to the build-up of plaque in arteries other than those that supply the heart or the brain, such as in the legs or arms.

If untreated, the consequences of such stenoses can be severe. For instance, a stenosis in a coronary artery due to CAD can result in coronary ischemia, i.e. a starvation of the blood supply to tissue, in this case to the heart muscle, ultimately resulting in the death of muscle cells. This condition is known as myocardial infarction, and is more commonly referred-to as a heart attack.

Current techniques for identifying stenoses in blood vessels include image-based techniques wherein medical images of the vasculature are analyzed in order to identify locations in which the blood vessel lumen is narrowed as compared to elsewhere in the lumen. The identified locations are then flagged for follow-up investigations. However, this approach has several drawbacks. Firstly, it relies heavily on accurate measurements of the blood vessel lumen. In practice, accurate measurements of the blood vessel lumen can be difficult to obtain from medical images due to confounding effects such as image noise, the limited spatial resolution of the imaging modality, image artifacts due to motion, calcifications, soft-plaques, and metal, and the limitations of step-and-shoot image acquisition techniques that are often used to acquire medical images. Secondly, not all stenoses are medically relevant, or actionable. For instance, the myocardial muscle which is supplied by the coronary vessels can cope with some stenoses without tangible damage. Consequently, current techniques for identifying stenoses in blood vessels can lead to a large proportion of false-positives, and this wastes time in unnecessary follow-up investigations.

Thus, there is a need for improvements in the identification of vascular stenoses.

### SUMMARY

According to one aspect of the present disclosure, a computer-implemented method of identifying a potential location of a stenosis in a vessel, is provided. The method includes:
receiving medical image data representing an anatomical region, the anatomical region including one or more vessels and adjoining tissue;
estimating, from the medical image data an actual perfusion map representing a spatial distribution of perfused blood in the adjoining tissue;
analyzing the medical image data to generate, for each of a plurality of candidate locations along the one or more vessels, an expected perfusion map representing a spatial distribution of perfused blood in the adjoining tissue as a result of blood flowing downstream of the candidate location;
calculating, for each of a plurality of the candidate locations, i) a correlation value representing a correlation between the expected perfusion map, and the actual perfusion map, or ii) a perfused blood value representing an amount of perfused blood in the actual perfusion map within an expected perfused blood boundary defined by a spatial extent of the perfused blood in the expected perfusion map; and
identifying one or more of the candidate locations as the potential location of the stenosis based on the correlation values, or the perfused blood values, respectively.

In the above method, medical image data representing an anatomical region including one or more vessels and adjoining tissue is used to estimate an actual perfusion map. The actual perfusion map represents a spatial distribution of perfused blood in the tissue adjoining the blood vessel(s). The medical image data is also used to generate an expected perfusion map for each of multiple candidate locations along a vessel. Each expected perfusion map represents an expected spatial distribution of perfused blood in the tissue surrounding the vessel as a result of blood flowing downstream of the candidate location.

In accordance with one aspect, each expected perfusion map is correlated with the actual perfusion map to provide a correlation value for the corresponding candidate location. One or more candidate locations are then identified as potential location of the stenosis based on the correlation values. In this aspect, the correlation values represent a likelihood of a candidate location being a location of a stenosis. Thus, candidate locations having relatively higher correlation values may be deemed to be indicative of a potential location of a stenosis, whereas candidate locations having relatively lower correlation values may be deemed not to be indicative of a potential location of a stenosis.

In accordance with another aspect, each expected perfusion map is used to calculate a perfused blood value for the corresponding candidate location. The perfused blood value represents an amount of perfused blood in the actual perfusion map within an expected perfused blood boundary defined by the spatial extent of the perfused blood in the expected perfusion map. One or more candidate locations are then identified as potential locations of the stenosis based on the perfused blood values. In this aspect, the magnitude of the gradient of the perfused blood values along the vessel represents a likelihood of a candidate location being a location of a stenosis. Thus, candidate locations in which there is a relatively higher magnitude of the gradient in the perfused blood values may be deemed to be indicative of a potential location of a stenosis, whereas candidate locations in which there is a relatively lower magnitude of the gradient in the perfused blood values may be deemed not to be indicative of a potential location of a stenosis.

The two aspects share the common approach of determining a potential location of a stenosis by assessing the expected perfusion maps for the candidate locations with respect to an actual perfusion map. Since this assessment is made on the basis of perfusion maps, i.e. spatial distributions of perfused blood, it does not require accurate measurements of the blood vessel lumen in the medical images. This, in-turn, reduces the proportion of false-positive identifications of stenoses, and consequently provides a more reliable technique for identifying the locations of stenoses.

Further aspects, features, and advantages of the present disclosure will become apparent from the following description of examples, which is made with reference to the accompanying drawings.

### BRIEF DESCRIPTION OF THE DRAWINGS

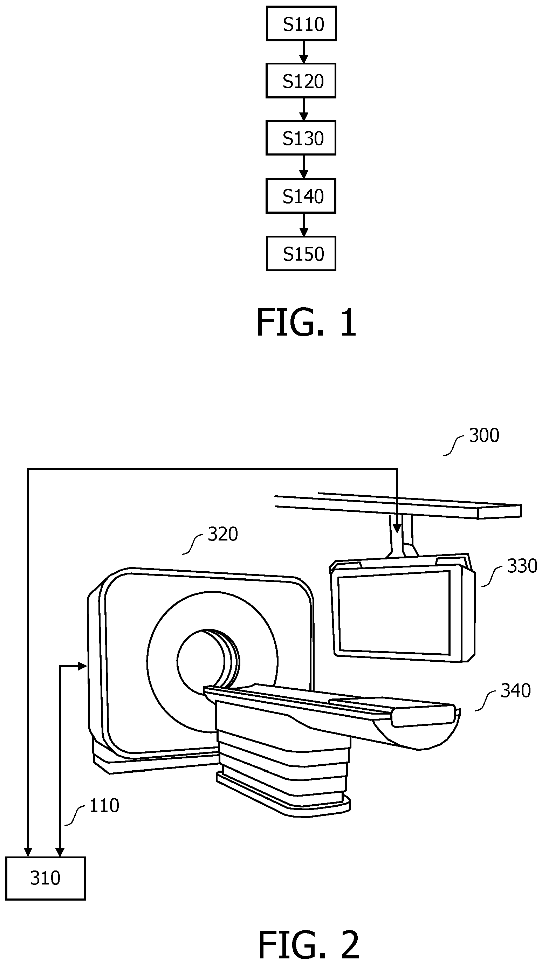

Fig. 1 is a flowchart illustrating an example of a computer-implemented method of identifying a potential location of a stenosis in a vessel, in accordance with some aspects of the present disclosure.
Fig. 2 is a schematic diagram illustrating an example of a system 300 for identifying a potential location of a stenosis in a vessel, in accordance with some aspects of the present disclosure.

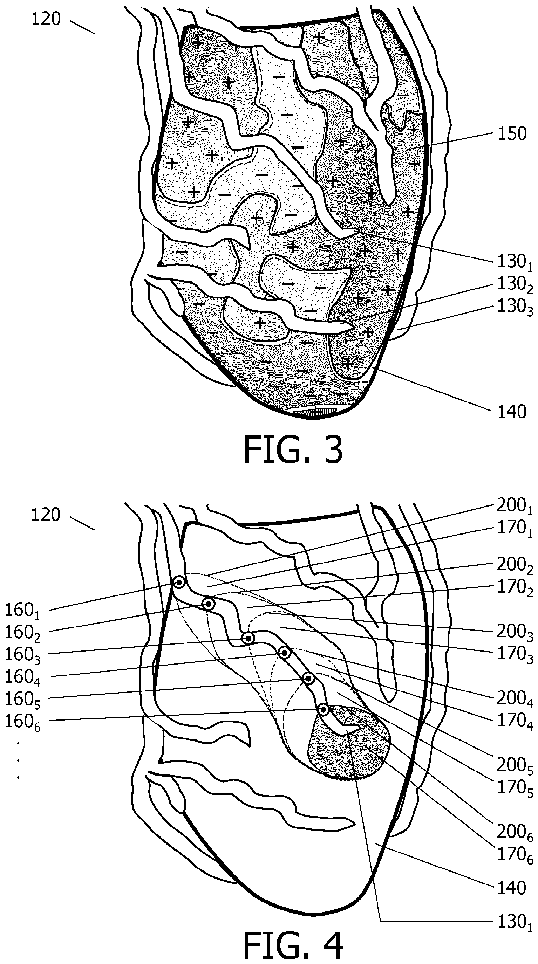

Fig. 3 illustrates an example of an actual perfusion map 150 for an anatomical region 120, in accordance with some aspects of the present disclosure.
Fig. 4 illustrates examples of expected perfusion maps 170_{1..6} for corresponding candidate locations 160_{1..6} along a vessel 130₁, in accordance with some aspects of the present disclosure.

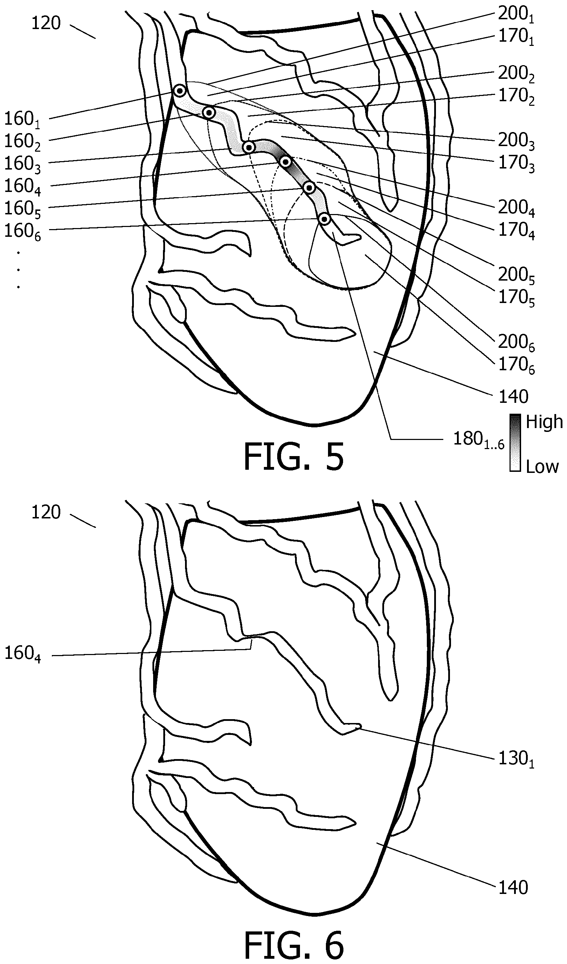

Fig. 5 illustrates an example of a graphical representation of correlation values 180_{1..6} that have been calculated for each of a plurality of candidate locations 160_{1..6} along a vessel, and wherein each correlation value is calculated by correlating the expected perfusion map 170_{1..6} for the candidate location 160_{1..6} with the actual perfusion map 150, in accordance with some aspects of the present disclosure.
Fig. 6 illustrates an example of a graphical representation of an anatomical region 120 including a vessel 130₁ and wherein the graphical representation of the vessel 130₁ has been adapted to indicate a potential location of a stenosis 160₄, in accordance with some aspects of the present disclosure.

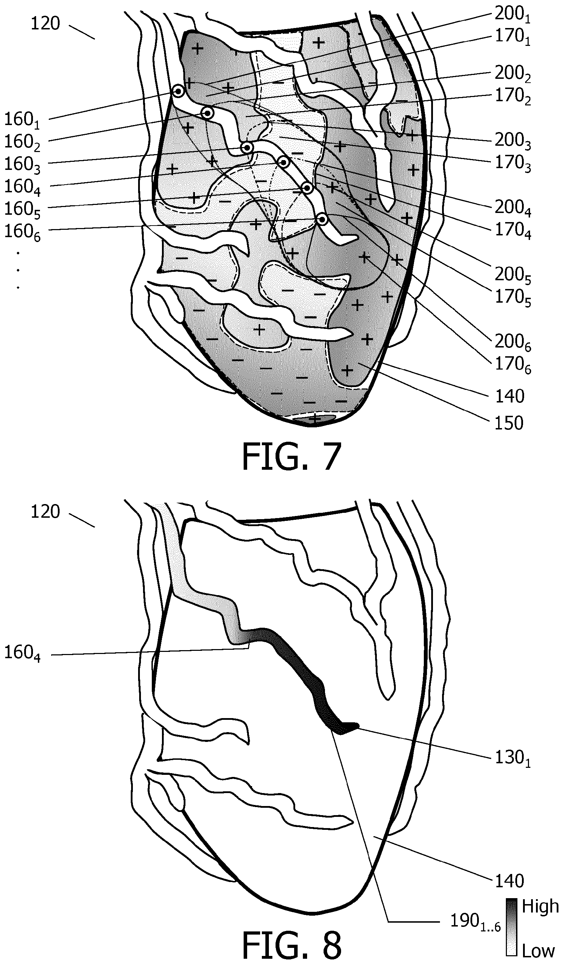

Fig. 7 illustrates examples of expected perfused blood boundaries 200_{1..6} for corresponding candidate locations 160_{1..6}, overlaid on an actual perfusion map 150, in accordance with some aspects of the present disclosure.
Fig. 8 illustrates an example of a graphical representation of an anatomical region 120 including a vessel 130₁ and wherein the graphical representation includes calculated perfused blood values 190_{1..6} for each of a plurality of candidate locations 160_{1..6} along a vessel 130₁, in accordance with some aspects of the present disclosure.

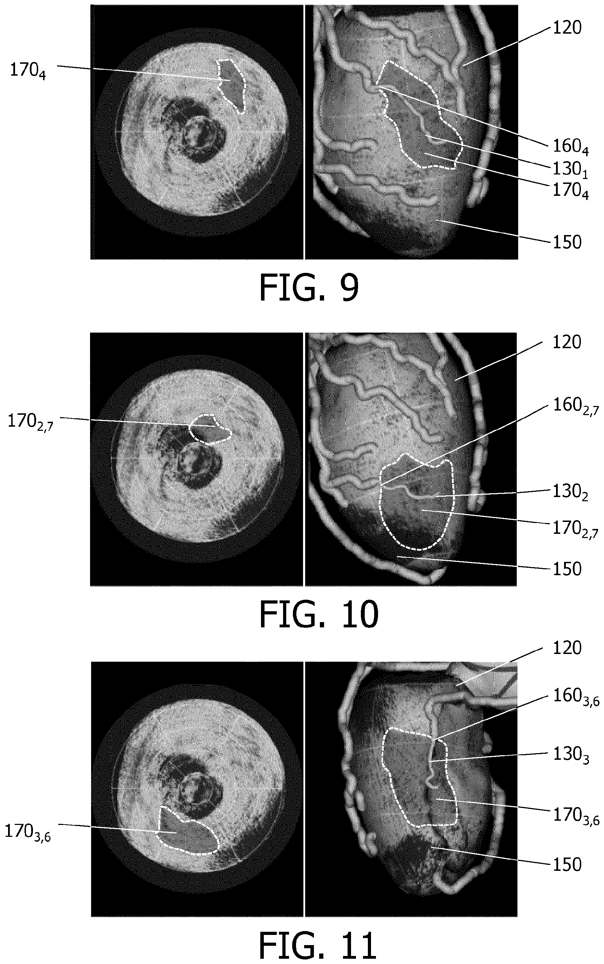

Fig. 9 illustrates an example of a graphical representation of an anatomical region 120 including a vessel 130₁, and wherein the graphical representation of the vessel 130₁ has been adapted to indicate a potential location 160₄ of a stenosis, in accordance with some aspects of the present disclosure.
Fig. 10 illustrates an example of a graphical representation of an anatomical region 120 including a vessel 130₂, and wherein the graphical representation of the vessel 130₂ has been adapted to indicate a potential location 160_{2,7} of a stenosis, in accordance with some aspects of the present disclosure.
Fig. 11 illustrates an example of a graphical representation of an anatomical region 120 including a vessel 130₃, and wherein the graphical representation of the vessel 130₃ has been adapted to indicate a potential location 160_{3,6} of a stenosis, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to a computer-implemented method of identifying a potential location of a stenosis in a vessel. In some examples, reference is made to a stenosis in an artery such as a coronary artery. However, it is to be appreciated that the method may also be used to identify potential locations of stenoses in arteries that are in other parts of the anatomy than the heart. For instance, the method may also be used to identify a potential location of a stenosis in an artery in the liver, lung, leg, thorax, eye, brain, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are performed in the computer-implemented methods disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations performed in the computer-implemented methods disclosed herein.

As mentioned above, there is a need for improvements in the identification of vascular stenoses.

Fig. 1 is a flowchart illustrating an example of a computer-implemented method of identifying a potential location of a stenosis in a vessel, in accordance with some aspects of the present disclosure. Fig. 2 is a schematic diagram illustrating an example of a system 300 for identifying a potential location of a stenosis in a vessel, in accordance with some aspects of the present disclosure. The system 300 illustrated in Fig. 2 includes one or more processors 310. It is noted that operations described as being performed in the method illustrated in Fig. 1, may also be performed by the one or more processors 310 of the system 300 illustrated in Fig. 2. Moreover, it is noted that operations described as being performed by the one or more processors illustrated in Fig. 2, may also be performed as part of the method illustrated in Fig. 1.

With reference to Fig. 1, the computer-implemented method of identifying a potential location of a stenosis in a vessel, includes:
receiving S110 medical image data 110 representing an anatomical region 120, the anatomical region including one or more vessels 130_{1..i} and adjoining tissue 140;
estimating S120, from the medical image data 110, an actual perfusion map 150 representing a spatial distribution of perfused blood in the adjoining tissue 140;
analyzing S130 the medical image data 110 to generate, for each of a plurality of candidate locations 160_{1..j} along the one or more vessels, an expected perfusion map 170_{1..j} representing a spatial distribution of perfused blood in the adjoining tissue 140 as a result of blood flowing downstream of the candidate location;
calculating S140, for each of a plurality of the candidate locations 160_{1..j}, i) a correlation value 180_{1..j} representing a correlation between the expected perfusion map 170_{1..j}, and the actual perfusion map 150, or ii) a perfused blood value 190_{1..j} representing an amount of perfused blood in the actual perfusion map 150 within an expected perfused blood boundary 200_{1..j} defined by a spatial extent of the perfused blood in the expected perfusion map 170_{1..j}; and
identifying S150 one or more of the candidate locations 160_{1..j} as the potential location of the stenosis based on the correlation values 180_{1..j}, or the perfused blood values 190_{1..j}, respectively.

In the above method, medical image data representing an anatomical region including one or more vessels and adjoining tissue is used to estimate an actual perfusion map. The actual perfusion map represents a spatial distribution of perfused blood in the tissue adjoining the blood vessel(s). The medical image data is also used to generate an expected perfusion map for each of multiple candidate locations along a vessel. Each expected perfusion map represents an expected spatial distribution of perfused blood in the tissue surrounding the vessel as a result of blood flowing downstream of the candidate location.

In accordance with one aspect, each expected perfusion map is correlated with the actual perfusion map to provide a correlation value for the corresponding candidate location. One or more candidate locations are then identified as potential location of the stenosis based on the correlation values. In this aspect, the correlation values represent a likelihood of a candidate location being a location of a stenosis. Thus, candidate locations having relatively higher correlation values may be deemed to be indicative of a potential location of a stenosis, whereas candidate locations having relatively lower correlation values may be deemed not to be indicative of a potential location of a stenosis.

In accordance with another aspect, each expected perfusion map is used to calculate a perfused blood value for the corresponding candidate location. The perfused blood value represents an amount of perfused blood in the actual perfusion map within an expected perfused blood boundary defined by the spatial extent of the perfused blood in the expected perfusion map. One or more candidate locations are then identified as potential locations of the stenosis based on the perfused blood values. In this aspect, the magnitude of the gradient of the perfused blood values along the vessel represents a likelihood of a candidate location being a location of a stenosis. Thus, candidate locations in which there is a relatively higher magnitude of the gradient in the perfused blood values may be deemed to be indicative of a potential location of a stenosis, whereas candidate locations in which there is a relatively lower magnitude of the gradient in the perfused blood values may be deemed not to be indicative of a potential location of a stenosis.

The two aspects share the common approach of determining a potential location of a stenosis by assessing the expected perfusion maps for the candidate locations with respect to an actual perfusion map. Since this assessment is made on the basis of perfusion maps, i.e. spatial distributions of perfused blood, it does not require accurate measurements of the blood vessel lumen in the medical images. This, in-turn, reduces the proportion of false-positive identifications of stenoses, and consequently provides a more reliable technique for identifying the locations of stenoses.

With reference to the method illustrated in Fig. 1, in the operation S110, medical image data 110 is received. The medical image data 110 represents an anatomical region 120. The anatomical region includes one or more vessels 130_{1..i} and adjoining tissue 140.

The medical image data 110 that is received in the operation S110 may be generated by various types of imaging systems. For instance, it may be generated by a volumetric imaging system such as a magnetic resonance imaging "MRI" system, or a computed tomography "CT" imaging system. Alternatively, it may be generated by two-dimensional imaging system such as a projection X-ray imaging system. In the example illustrated in Fig. 2, the medical image data 110 is generated by a CT imaging system 320. In examples in which the medical image data 110 is generated by a CT, or a projection X-ray, imaging system, the imaging system may be spectral X-ray imaging system. In other words, the medical image data 110 may be generated by a spectral CT imaging system, or a spectral X-ray projection imaging system. Spectral X-ray imaging systems generate X-ray attenuation data representing X-ray attenuation within multiple different energy intervals. The X-ray attenuation data from spectral X-ray imaging systems may be processed in order to distinguish between media that have similar X-ray attenuation values when measured within a single energy interval, and which would be indistinguishable in conventional X-ray attenuation data. Thus, the X-ray attenuation data from spectral X-ray imaging systems may be used to provide images with improved specificity to materials such as contrast agent, tissue, bone, and so forth.

In general, the medical image data 110 that is received in the operation 5110 may represent a single static image, or it may represent a temporal sequence of images, i.e. fluoroscopic images. In some examples, the medical image data 110 may be generated instantaneously before being received by the one or more processors 310. For instance, the medical image data 110 may represent a live sequence of images. Alternatively, the medical image data 110 may have been generated some seconds, minutes, hours, or even days, or a longer period, beforehand. In the latter case, the medical image data 110 may have been generated prior to performing a medical procedure on the anatomical region. In this case, the medical image data 110 may be referred-to as pre-procedural medical image data.

In some examples, the medical image data 110 that is received in the operation S110 is generated subsequent to the injection of a contrast agent into the vasculature of a subject. Medical image data 110 that is generated by a CT imaging system, or by a projection X-ray imaging system, may be generated in this manner, for example. The contrast agent highlights blood in the medical image data. In some examples, the medical image data 110 may represent digital subtraction angiographic "DSA" images. DSA images are generated by subtracting the image intensity values of an X-ray image that has been generated prior to the injection of a contrast agent into the vasculature, from the image intensity values in corresponding positions in X-ray images that are generated after the contrast agent has been injected. Thus, DSA images provide a visualization of blood in the anatomy in the absence of confounding image features such as bone, and which are common to the images before and after contrast agent injection.

The medical image data 110 that is received in the operation S110 may be received from various sources, including from a medical imaging system such as the imaging systems described above. Alternatively, the medical image data 110 may be received from another source, such as a database, a computer readable storage medium, the internet, the cloud, and so forth. The medical image data 110 may be received via any form of data communication, including wired and wireless communication. By way of some examples, when wired communication is used, the communication may take place via an electrical or optical cable, and when wireless communication is used, the communication may for example be via RF or infrared signals.

The medical image data 110 that is received in the operation S110 represents an anatomical region 120. The anatomical region includes one or more vessels 130_{1..i} and adjoining tissue 140. In the examples that are described below, the anatomical region is the heart, and the one or more vessels 130_{1..i} are coronary arteries. In these examples, the adjoining tissue may include tissue such as myocardial tissue that adjoins, or in other words is connected to, the vessel(s) and which is consequently perfused by blood flowing in the one or more vessels 130_{1..i}. In general, however, the anatomical region 120 may be any anatomical region, and the vessels may be any type of artery. Thus, in other examples, other types of tissue than myocardial tissue may be perfused by the blood flowing in the one or more vessels 130_{1..i}.

Returning to the method illustrated in Fig. 1, in the operation S120, an actual perfusion map 150 is estimated from the medical image data 110. The actual perfusion map 150 represents a spatial distribution of perfused blood in the tissue adjoining the one or more vessels 130_{1..i}.

In some examples, the medical image data 110 is acquired subsequent to the injection of a contrast agent into the vasculature of a subject. For example, if the medical image data is generated by a CT, projection X-ray, or MRI imaging system, a contrast agent that includes a substance such as iodine, of gadolinium, may be injected into the vasculature prior to acquisition of the medical image data 110. The contrast agent provides enhanced visibility of the blood in the tissue, and consequently the image intensity values in the tissue may be used as surrogate values for the amount of perfused blood in the adjoining tissue. In these examples, the medical image data 110 that is received in the operation 5110 represents an actual distribution of a contrast agent in the anatomical region 120, and in the operation S120, the actual perfusion map 150 is estimated based on the actual distribution of the contrast agent in the anatomical region.

In other examples the medical image data 110 is acquired without the use of a contrast agent. For instance, medical image data may be acquired from an MRI imaging system, and techniques such as arterial spin labelling "ASL", and other diffusion MRI models, may be used to provide a perfusion map in the absence of a contrast agent. In these examples, the actual perfusion map 150 is estimated from the medical image data 110 using a diffusion model.

Fig. 3 illustrates an example of an actual perfusion map 150 for an anatomical region 120, in accordance with some aspects of the present disclosure. In Fig. 3, the anatomical region 120 is the heart, and the one or more vessels 130_{1..i} represent coronary arteries. The coronary arteries 130_{1..i} supply oxygen-rich blood to the adjoining tissue 140 in a process known as perfusion. This gives rise to a spatial distribution of perfused blood in the adjoining tissue 140 (e.g. myocardial tissue, a type of muscle tissue) and facilitates the pumping of the heart.

The actual perfusion map 150 in the example illustrated in Fig. 3 is obtained by acquiring medical image data 110 representing the heart using a CT imaging system subsequent to the injection of a contrast agent into the vasculature. The image intensity values in the actual distribution of the contrast agent in the anatomical region have been used as surrogate values for the amount of perfused blood in the adjoining tissue. In the example illustrated in Fig. 3, the spatial distribution of perfused blood in the adjoining tissue has been normalized by subtracting from the image intensity values in the actual distribution of the contrast agent, a median value for the image intensity in myocardial tissue. This results in the positive values (hyper-perfused relative to the median value) and negative values (hypo-perfused relative to the median value) in the spatial distribution that are within the respective boundaries labelled as "+" and "-"in Fig. 3. The magnitude of hyper-, or hypo-perfusion at each point in the spatial distribution is illustrated by way of the grayscale intensity in the actual perfusion map 150. Thus, dark-shaded areas within boundaries labelled as "+" correspond to relatively greater degree of hyper-perfusion than light-shaded area within boundaries labelled as "+". Similarly, dark-shaded areas within boundaries labelled as "-" correspond to relatively lesser degree of hypo-perfusion than light-shaded areas within boundaries labelled as "-".

Returning to the method illustrated in Fig. 1, in the operation S130, the medical image data 110 is analyzed to generate, for each of a plurality of candidate locations 160_{1..j} along the one or more vessels, an expected perfusion map 170_{1..j} representing a spatial distribution of perfused blood in the adjoining tissue 140 as a result of blood flowing downstream of the candidate location. This operation is described with reference to Fig. 4, which illustrates examples of expected perfusion maps 170_{1..6} for corresponding candidate locations 160_{1..6} along a vessel 130₁, in accordance with some aspects of the present disclosure.

In general, the generation of the expected perfusion maps 170_{1..j} in the operation S130 involves identifying a path of the one or more vessels 130_{1..i} in the anatomical region 120, defining a plurality of candidate locations 160_{1..j} along the path of the one or more vessels 130_{1..i}, and generating the expected perfusion map 170_{1..j} by modelling the spatial distribution of perfused blood in the adjoining tissue 140 as a result of blood flowing downstream of each candidate location 160_{1..j}.

In the operation S130, the path(s) of the one or more vessels 130_{1..i} may be identified using various techniques. One technique involves segmenting the medical image data 110. Various known segmentation techniques may be used for this purpose, including region growing, thresholding, feature detection, model-based segmentation, and segmentation using a trained neural network such as a U-Net. Such segmentation techniques may be used to segment the lumen(s) of the vessel(s) 130_{1..i} in the medical image data 110, and to thereby identify the path(s) of the vessels(s) 130_{1..i}. In some examples, the path corresponds to the centerline(s) of the vessel(s) 130_{1..i}. The centerline(s) of the vessel(s) may be identified by determining positions along the vessel(s) that are mid-way between its bilateral borders. Another technique for identifying the path in the operation S130 involves analyzing the medical image data 110 using a neural network trained to identify the path. In this technique, a neural network such as a convolutional neural network "CNN" may be trained to identify the path(s) of vessels by training the neural network using training data that includes multiple medical images representing anatomical regions that include vessels, and corresponding ground truth data representing delineated paths of the vessels. Another technique for identifying the path in the operation S130 involves using a path-finding algorithm such as Fast Marching. A dynamic programming technique may be used to optimize the position of the centerline with respect to the borders of the lumen.

It is noted that in these example techniques, the identification of the path of the vessel(s) does not require accurate measurements of the blood vessel lumen in the medical images. This improves their resilience to confounding effects such as image noise, the limited spatial resolution of the imaging modality, image artifacts due to motion, calcifications, soft-plaques, and metal, and the limitations of step-and-shoot image acquisition techniques that are often used to acquire medical images.

Having identified the path(s) of the one or more vessels 130_{1..i}, a plurality of candidate locations 160_{1..j} are then defined along the path of the one or more vessels 130_{1..i}. The candidate locations may have any desired distribution along the vessel(s). In the example illustrated in Fig. 4, six candidate locations are illustrated with regular spacing, i.e. candidate locations 160_{1..6}. In general there may be any number of candidate locations, and the candidate locations may have any desired spacing. The spacing may be regular, or irregular. In one example, the candidate locations are defined at adjacent pixels in the medical image data 110. In general, a higher density of candidate locations 160_{1..j} along the vessel(s) provides a more accurate identification of a potential location of a stenosis at the expense of increased processing time.

Having defined the candidate locations 160_{1..j}, for each candidate location 160_{1..j}, an expected perfusion map 170_{1..j} representing a spatial distribution of perfused blood in the adjoining tissue 140 as a result of blood flowing downstream of the candidate location, is then generated. Two approaches for this operation are described below.

In one approach, the expected perfusion maps 170_{1..j} are generated by modelling the spatial distribution of perfused blood in the adjoining tissue 140 as a result of blood flowing downstream of the candidate location in the absence of a stenosis in the one or more vessels 130_{1..i}. This approach models "normal" perfusion of the adjoining tissue 140 by the one or more vessels 130_{1..i}. In one example of this approach, values representing the spatial distribution are assigned to the adjoining tissue 140 downstream of the candidate location based on a distance of the tissue from the path. In one implementation, pixels/ voxels representing adjoining tissue in the anatomical region are assigned to their nearest path, and a non-zero value is assigned to the tissue pixel/ voxel if it is downstream of the corresponding candidate location, and a zero value is assigned to the pixel/ voxel if it is upstream of the candidate location. This implementation is illustrated in Fig. 4. With reference to Fig. 4, the expected perfusion map 170₆ corresponding to the candidate location 160₆ for the vessel 130₁, in the expected perfusion map 170₆, tissue pixels/ voxels that are deemed to be closest to the path of the vessel 130₁ and which are downstream of the candidate location 160₆ have been assigned a value of unity, as indicated by the dark-shaded region within the boundary 200₆. In the expected perfusion map 170₆, pixels/ voxels that are outside of the boundary 200₆ have been assigned a value of zero, as indicated by the light-shaded region outside the boundary 200₆. The boundaries 200_{1..5} of the expected perfusion maps 170_{1..5} for the corresponding candidate locations 160_{1..5}, are also illustrated in Fig. 4. The pixel/ voxel values within these boundaries are likewise unity within their respective boundaries, and zero outside their respective boundaries. For the purpose of illustration, the expected perfusion maps 170_{1..5} have not been shaded within their boundaries in order to avoid obscuring the expected perfusion map 170₆.

Instead of assigning values to the tissue pixels/ voxels in the manner described above, the expected perfusion maps 170_{1..j} may be generated by assigning values to the tissue pixels/ voxels in other ways. These include assigning non-binary, or in other words, analogue values, to the tissue pixels/ voxels. For instance, in another implementation, a reference spatial distribution of perfused blood may be assigned to the adjoining tissue 140. The reference spatial distribution may be assigned to the adjoining tissue 140 based on factors such as population data, left versus right coronary artery dominance, a coronary atlas, and so forth. In yet another example, the spatial distribution of perfused blood in the adjoining tissue 140 may be modelled using a parametric perfusion model.

In another approach, the expected perfusion maps 170_{1..j} are generated by modelling the spatial distribution of perfused blood in the adjoining tissue 140 as a result of blood flowing downstream of the candidate location in the presence of a candidate stenosis in the candidate location. In this approach, the spatial distribution of perfused blood in the adjoining tissue 140 as a result of blood flowing downstream of the candidate location in the presence of a candidate stenosis in the candidate location, is modelled using a parametric perfusion model in which the parameters represent a degree of stenosis at the candidate location along the vessel path. An example of such a model is described in a document by Boileau, E., et al., "Estimating the accuracy of a reduced-order model for the calculation of fractional flow reserve (FFR)", Volume34, Issue1, January 2018, e2908. In this approach, the operations of generating the expected perfusion map 170_{1..j}, and the calculating operation S140 described below, may be performed iteratively for a plurality of different magnitudes of the candidate stenosis, to further determine a magnitude of the stenosis at the candidate location in the vessel.

Since in both of these approaches, the generation of the expected perfusion maps 170_{1..j} does not require accurate measurements of the blood vessel lumen in the medical images, they are more resilient to confounding effects such as image noise, the limited spatial resolution of the imaging modality, image artifacts due to motion, calcifications, soft-plaques, and metal, and the limitations of step-and-shoot image acquisition techniques that are often used to acquire medical images.

Returning to the method illustrated in Fig. 1, in the operation S140, for each of a plurality of the candidate locations 160_{1..j}, i) a correlation value 180_{1..j} is calculated. The correlation value represents a correlation between the expected perfusion map 170_{1..j}, and the actual perfusion map 150. In the corresponding operation S150, one or more of the candidate locations 160_{1..j} is identified as the potential location of the stenosis based on the correlation values 180_{1..j}. Alternatively, in the operation S140, for each of a plurality of the candidate locations 160_{1..j}, ii) a perfused blood value 190_{1..j} is calculated. For each candidate location 160_{1..j}, the perfused blood value represents an amount of perfused blood in the actual perfusion map 150 within an expected perfused blood boundary 200_{1..j} defined by a spatial extent of the perfused blood in the expected perfusion map 170_{1..j}. In the corresponding operation S150, one or more of the candidate locations 160_{1..j} is identified as the potential location of the stenosis based on the perfused blood values 190_{1..j}. The first of these options, i) is described with reference to Fig. 5 and Fig. 6, and the second of these options, ii) is described with reference to Fig. 7 and Fig. 8.

In the first of these options, i) a correlation value 180_{1..j} is calculated for each candidate location 160_{1..j} in the operation S140, and in the corresponding operation S150, one or more of the candidate locations 160_{1..j} is identified as the potential location of the stenosis based on the correlation values 180_{1..j}. The correlation values 180_{1..6} in this option represent a likelihood of a candidate location being a location of a stenosis. Thus, candidate locations having relatively higher correlation values are deemed to be indicative of a potential location of a stenosis, whereas candidate locations having relatively lower correlation values are deemed not to be indicative of a potential location of a stenosis.

The calculation of the correlation values 180_{1..j} in accordance with this option is now described with reference to Fig. 5, which illustrates an example of a graphical representation of correlation values 180_{1..6} that have been calculated for each of a plurality of candidate locations 160_{1..6} along a vessel, and wherein each correlation value is calculated by correlating the expected perfusion map 170_{1..6} for the candidate location 160_{1..6} with the actual perfusion map 150, in accordance with some aspects of the present disclosure. By way of an example, the correlation value 180₁ for the candidate location 160₁ is determined by correlating the expected perfusion map 170₁ in Fig. 5, with the actual perfusion map 150 illustrated in Fig. 3. The correlation values 180_{1..j} may be calculated using various mathematical functions, such as by evaluating a correlation coefficient, or by evaluating a covariance. In Fig. 5, the resulting correlation values 180_{1..6} corresponding to the candidate locations 160_{1..6} are illustrated via the grayscale intensity of the vessel 130₁ in Fig. 5. Relatively higher correlation values are represented by darker-shaded areas, and relatively-lower correlation values are represented by lighter-shaded areas. In the example illustrated in Fig. 5, the candidate location 160₁ is illustrated in the graphical representation of the vessel 130₁ via a relatively lighter-shaded lumen than the candidate location 160₄, and consequently the candidate location 160₄ indicates a more likely location of a stenosis than the candidate location 160₁.

The graphical representation of the correlation values 180_{1..6} illustrated in Fig. 5 is one example the operation of identifying S150 one or more of the candidate locations 160_{1..j} as the potential location of the stenosis based on the correlation values 180_{1..j}. Alternatively, or additionally, one or more of the candidate locations 160_{1..j} having the highest correlation values 180_{1..j} may be identified as the potential location of the stenosis in the operation S150. For instance, a graphical representation of the anatomical region including the one or more vessels 130_{1..i} and the adjoining tissue 140, may be outputted, and a marker may be placed at the candidate location having the highest correlation value in order to identify the most likely location of the stenosis.

Another example of the operation of identifying S150 one or more of the candidate locations 160_{1..j} as the potential location of the stenosis based on the correlation values 180_{1..j} is illustrated in Fig. 6. Fig. 6 illustrates an example of a graphical representation of an anatomical region 120 including a vessel 130₁ and wherein the graphical representation of the vessel 130₁ has been adapted to indicate a potential location of a stenosis 160₄, in accordance with some aspects of the present disclosure. In the example illustrated in Fig. 6, the diameter of the vessel 130₁ has been artificially reduced in the vicinity of the candidate location 160₄ in order to highlight its potential location. The graphical representation of the one or more vessels 130_{1..i} may alternatively be adapted in other ways to indicate a potential location of a stenosis. For instance, the vessel may be highlighted with a color, or a marker, or using another technique. More generally, the operation of identifying S150 one or more of the candidate locations 160_{1..j} as the potential location of the stenosis, may include:
outputting a graphical representation of the anatomical region 120 including the one or more vessels 130_{1..i} and the adjoining tissue 140; and
displaying, in the graphical representation, one or more markers indicating the one or more candidate locations 160_{1..j} having the highest correlation values 180_{1..j}, or the one or more candidate locations 160_{1..j} corresponding to a local maximum in a magnitude of a gradient of the perfused blood values 190_{1..j};
   and/or
adapting the graphical representation of the one or more vessels 130_{1..i} to indicate the identified potential location of the stenosis;
   and/or
displaying in the graphical representation, a ranked list comprising the one or more candidate locations 160_{1..j} identified as the potential location of the stenosis.

In these examples, the magnitude of the correlation value at the identified potential location of the stenosis may also be outputted. This helps to convey to a user the level of confidence in the assessment. The graphical representation of the anatomical region 120 may also be overlaid with the actual perfusion map 150. This provides additional context to the basis on which the potential location of the stenosis has been identified.

As mentioned above, another option is also provided for the operation S140 illustrated in Fig. 1. In this second option ii) a perfused blood value 190_{1..j} is calculated for each of the candidate locations 160_{1..j}. For each candidate location 160_{1..j}, the perfused blood value represents an amount of perfused blood in the actual perfusion map 150 within an expected perfused blood boundary 200_{1..j} defined by a spatial extent of the perfused blood in the expected perfusion map 170_{1..j}. In the corresponding operation S150, one or more of the candidate locations 160_{1..j} are identified as the potential location of the stenosis based on the perfused blood values 190_{1..j}.

In this second option, the magnitude of the gradient of the perfused blood values 190_{1..j} along the vessel represents a likelihood of a candidate location being a location of a stenosis. Candidate locations in which there is a relatively higher magnitude of the gradient in the perfused blood values are deemed to be indicative of a potential location of a stenosis, whereas candidate locations in which there is a relatively lower magnitude of the gradient in the perfused blood values are deemed not to be indicative of a potential location of a stenosis.

In this second option, the magnitude of the gradient of the perfused blood values along the vessel may be observed from a graphical representation of the perfused blood values along the vessel. Candidate locations in which the magnitude of the gradient is relatively higher appear as an abrupt transition in the perfused blood values. Alternatively, a value for the magnitude of the gradient may be calculated from the perfused blood values and its value may be outputted. Thus, in this option, in the operation S150, one or more of the candidate locations 160_{1..j} may be identified as the potential location of the stenosis by outputting a graphical representation of the perfused blood values 190_{1..j}, or by calculating, and outputting, the magnitude of the gradient of the perfused blood values. In one example, a gradient of the perfused blood values 190_{1..j} along the vessel, is calculated, and one or more of the candidate locations 160_{1..j} corresponding to a local maximum in a magnitude of the gradient of the perfused blood values 190_{1..j} are identified as the potential location of the stenosis in the operation S150.

The calculation of the perfused blood values 190_{1..j} in accordance with this second option is now described with reference to Fig. 7, which illustrates examples of expected perfused blood boundaries 200_{1..6} for corresponding candidate locations 160_{1..6}, overlaid on an actual perfusion map 150, in accordance with some aspects of the present disclosure. In this second option, two alternative techniques are described for calculating the perfused blood values 190_{1..j} in the operation S140. In one example technique, the perfused blood values 190_{1..j} are calculated for each of the candidate locations 160_{1..j} by:
determining the expected perfused blood boundary 200_{1..j} based on a spatial extent of the perfused blood in the expected perfusion map 170_{1..j};
mapping the expected perfused blood boundary 200_{1..j} onto the actual perfusion map 150; and
analyzing the spatial distribution of perfused blood in the actual perfusion map 150 within the expected perfused blood boundary 200_{1..j}, to provide the perfused blood value 190_{1..j}.

With reference to Fig. 7, in this second option, the expected perfusion maps 170_{1..j} are determined using the techniques described above. The spatial extent of the perfused blood in the expected perfusion map 170_{1..j} is then used the determine the expected perfused blood boundary 200_{1..j}. With reference to the example candidate location 160₆, this may be performed by applying a threshold value to the spatial distribution values in the expected perfusion map 170₆. In the expected perfusion maps 170_{1..j} illustrated in Fig. 7, the spatial distribution values are either unity, or zero, as described with reference to Fig. 4, and so the perfused blood boundary 200₆ may be defined by applying a threshold value of e.g. 0.5 to the spatial distribution values in the expected perfusion map 170₆. In other expected perfusion maps, the spatial distribution values may be different to zero, and unity, and therefore other threshold values may be applied in this operation.

Having determined the expected perfused blood boundaries 200_{1..j}, these are then mapped onto the actual perfusion map 150, as illustrated in Fig. 7. The spatial distribution of perfused blood in the actual perfusion map 150 within the expected perfused blood boundary 200₆, is then analyzed to provide the perfused blood value 190₆ for the candidate location 160₆. Each perfused blood value, e.g. the perfused blood value 190₆ for the candidate location 160₆, may be calculated as a total, or an average of the values in the spatial distribution within the corresponding expected perfused blood boundary, e.g. the expected perfused blood boundary 200₆. For instance, each perfused blood value may be calculated as a mean value, or a mode, or a median, of the spatial distribution values within the expected perfused blood boundary. These operations are performed for each of the candidate locations illustrated in Fig. 7, resulting in perfused blood values 190_{1..6} corresponding to each of the candidate locations 160_{1..6}.

As described above, in this second option, the magnitude of the gradient of the perfused blood values along the vessel represents a likelihood of a candidate location being a location of a stenosis. Thus, candidate locations in which there is a relatively higher magnitude of the gradient in the perfused blood values may be deemed to be indicative of a potential location of a stenosis, whereas candidate locations in which there is a relatively lower magnitude of the gradient in the perfused blood values may be deemed not to be indicative of a potential location of a stenosis.

Thus, in this implementation, the potential location of the stenosis may be identified by:
outputting a graphical representation of the one or more vessels 130_{1..i}; and
wherein the operation of identifying S150 one or more of the candidate locations 160_{1..j} as the potential location of the stenosis based on the perfused blood values 190_{1..j}, comprises:
   a) indicating the perfused blood values 190_{1..j} in the graphical representation at the corresponding candidate locations 160_{1..j}; and/or
   b) calculating a gradient of the perfused blood values 190_{1..j} along the vessel, and identifying one or more of the candidate locations 160_{1..j} as the potential location of the stenosis based on a magnitude of the gradient of the perfused blood values 190_{1..j}.

An example of the first of these options is illustrated in Fig. 8, which illustrates an example of a graphical representation of an anatomical region 120 including a vessel 130₁ and wherein the graphical representation includes calculated perfused blood values 190_{1..6} for each of a plurality of candidate locations 160_{1..6} along a vessel 130₁, in accordance with some aspects of the present disclosure. In the example illustrated in Fig. 8, the perfused blood values 190_{1..6} are simply overlaid onto the graphical representation of the vessel 130₁. In this example, the perfused blood values 190_{1..6} are indicated by shading the vessel 130₁ in grayscale, relatively higher values being in relatively darker-shaded, and relatively lower values being relatively lighter-shaded. An observer may simply see the magnitude of the gradient of the perfused blood values 190_{1..6} in Fig. 8. For instance, the transition from lighter to darker shaded region at the candidate locations 160₄, indicates that this is the most likely location of the stenosis.

Instead of using the graphical representation illustrated in Fig. 8, the operation of identifying S150 one or more of the candidate locations 160_{1..j} as the potential location of the stenosis based on the perfused blood values 190_{1..j} may include by b) calculating a gradient of the perfused blood values 190_{1..j} along the vessel, and identifying one or more of the candidate locations 160_{1..j} as the potential location of the stenosis based on a magnitude of the gradient of the perfused blood values 190_{1..j}. In this example the value of the magnitude of the gradient may simply be outputted. For instance, the value of the magnitude of the gradient may be outputted in the form of a table for each candidate location, or it may be overlaid in the form of a color, or another graphical representation, onto a graphical representation of the vessel 130₁, such as the graphical representation illustrated in Fig. 8.

In another example technique, the perfused blood values 190_{1..j} are calculated for each of the candidate locations 160_{1..j} by:
mapping the expected perfusion map 170_{1..j} onto the actual perfusion map 150 to define the expected perfused blood boundary 200_{1..j} in the actual perfusion map;
weighting the spatial distribution of perfused blood in the actual perfusion map 150 within the expected perfused blood boundary 200_{1..j}, with the spatial distribution of perfused blood in the expected perfusion map 170_{1..j}; and
analyzing the weighted spatial distribution to provide the perfused blood value 190_{1..j}.

With reference to Fig. 7, in this technique, the expected perfusion maps 170_{1..j} are determined using the techniques described above. Each expected perfusion map is then mapped 170_{1..j} onto the actual perfusion map 150 to define the expected perfused blood boundary 200_{1..j}. Some of the expected perfusion maps described above have a well-defined boundary. For instance, expected perfusion maps such as the example perfusion maps 170₆ illustrated in Fig. 4, and in which the values in the spatial distribution are binary, have a well-defined boundary. In other expected perfusion maps, the values in the spatial distribution may have analogue values. The boundaries of such maps are therefore diffuse. The present example addresses this latter type of expected perfusion map by defining the expected blood boundary as the maximum extent of these non-zero analogue values. In accordance with this example, the spatial distribution of perfused blood in the actual perfusion map 150 within the expected perfused blood boundary 200_{1..j}, is weighted with the spatial distribution of perfused blood in the expected perfusion map 170_{1..j}. Pixels, or voxels, in the expected perfusion maps 170_{1..j} that are close to the expected perfused blood boundary 200_{1..j}, are likely to have relatively lower values than pixels or voxels that are close to a vessel. Consequently, the weighting that is applied in this operation places less emphasis on pixels, or voxels, in the expected perfusion maps 170_{1..j} that are close to the expected perfused blood boundary 200_{1..j} than those that are close to a vessel. The weighted spatial distribution is then analyzed in a similar manner to that described above to provide the perfused blood value 190_{1..j} for the corresponding location. This operation may be performed in the same manner as described above, i.e. by calculating a total, or an average value, e.g. a mean value, or a mode, or a median value, from the weighted values for the individual voxels within the corresponding expected perfused blood boundary 200_{1..j}. These operations are performed using the expected perfusion maps corresponding to each of the candidate locations illustrated in Fig. 7, resulting in perfused blood values 190_{1..6} for each of the candidate locations 160_{1..6}. The perfused blood values 190_{1..6} are then used to identify the potential location of the stenosis in the same manner as described above, i.e. by:
outputting a graphical representation of the one or more vessels 130_{1..i}; and
wherein the operation of identifying S150 one or more of the candidate locations 160_{1..j} as the potential location of the stenosis based on the perfused blood values 190_{1..j}, comprises:
   a) indicating the perfused blood values 190_{1..j} in the graphical representation at the corresponding candidate locations 160_{1..j}; and/or
   b) calculating a gradient of the perfused blood values 190_{1..j} along the vessel, and identifying one or more of the candidate locations 160_{1..j} as the potential location of the stenosis based on a magnitude of the gradient of the perfused blood values 190_{1..j}.

Some alternative examples of graphical representations of the anatomical region 120 and wherein the graphical representation of the vessel has been adapted to indicate a potential location of a stenosis, are illustrated in Fig. 9 - Fig. 11. These may be used with the implementations described with reference to Fig. 5 and Fig. 6 and in which a correlation values are calculated, as well as with the implementations described with reference to Fig. 7 and Fig. 8 and in which perfused blood values are calculated. In these examples, the diameter of the vessel has been artificially reduced downstream of the identified potential location of the stenosis in order to highlight its location. These graphical representations may also be outputted in the operation S150 instead of, or in addition to, the graphical representations in the examples provided above.

Fig. 9 illustrates an example of a graphical representation of an anatomical region 120 including a vessel 130₁, and wherein the graphical representation of the vessel 130₁ has been adapted to indicate a potential location 160₄ of a stenosis, in accordance with some aspects of the present disclosure. In this example, the diameter of the vessel 130₁ has been artificially reduced downstream of the identified potential location of the stenosis in order to highlight its location. In this example, the anatomical region 120 is overlaid with an actual perfusion map 150 in order to provide further context to the identified location of the stenosis. The expected perfusion map 170₄ for the candidate location 160₄ is also delineated in Fig. 10 by white dashed lines. The right-hand view of Fig. 9 represents a three-dimensional visualization of the anatomical region, similar to that in Fig. 3 - Fig. 8. The left-hand view of Fig. 9 represents a "bulls eye" view of the anatomical region that assists in visualizing the expected perfusion map 170₄ and the identified location of the stenosis.

Fig. 10 illustrates an example of a graphical representation of an anatomical region 120 including a vessel 130₂, and wherein the graphical representation of the vessel 130₂ has been adapted to indicate a potential location 160_{2,7} of a stenosis, in accordance with some aspects of the present disclosure. In this example, the diameter of the vessel 130₂ has been artificially reduced downstream of the identified potential location of the stenosis in order to highlight its location. In this example, the anatomical region 120 is overlaid with an actual perfusion map 150 in order to provide further context to the identified location of the stenosis. The expected perfusion map 170_{2,7} for the candidate location 160_{2,7} is also delineated in Fig. 10 by white dashed lines. The right-hand view of Fig. 10 represents a three-dimensional visualization of the anatomical region, similar to that in Fig. 3 - Fig. 8. The left-hand view of Fig. 10 represents a "bulls eye" view of the anatomical region that assists in visualizing the expected perfusion map 170_{2,7} and the identified location of the stenosis.

Fig. 11 illustrates an example of a graphical representation of an anatomical region 120 including a vessel 130₃, and wherein the graphical representation of the vessel 130₃ has been adapted to indicate a potential location 160_{3,6} of a stenosis, in accordance with some aspects of the present disclosure. In this example, the diameter of the vessel 130₂ has been artificially reduced downstream of the identified potential location of the stenosis in order to highlight its location. In this example, the anatomical region 120 is overlaid with an actual perfusion map 150 in order to provide further context to the identified location of the stenosis. The expected perfusion map 170_{3,6} for the candidate location 160_{3,6} is also delineated in Fig. 10 by white dashed lines. The right-hand view of Fig. 11 represents a three-dimensional visualization of the anatomical region, similar to that in Fig. 3 - Fig. 8. The left-hand view of Fig. 11 represents a "bulls eye" view of the anatomical region that assists in visualizing the expected perfusion map 170_{3,6} and the identified location of the stenosis.

In another example, the method described with reference to Fig. 1 is performed using segmented medical image data 110. In this example, method further described with reference to Fig. 1 includes segmenting the medical image data 110 to identify the adjoining tissue 140. The operations of estimating an actual perfusion map 150, and analyzing S130 the medical image data 110, are then performed using the segmented medical image data. Various segmentation techniques may be used for this purpose, including region growing, thresholding, feature detection, model-based segmentation, and segmentation using a trained neural network such as a CNN, or a U-Net.

In another example, the spatial distribution of perfused blood in the expected perfusion map 170_{1..j} and/or the actual perfusion map 150, is normalized. In this example, the method described with reference to Fig. 1 includes calculating S140, for each of a plurality of the candidate locations 160_{1..j}, i) a correlation value 180_{1..j} representing a correlation between the expected perfusion map 170_{1..j}, and the actual perfusion map 150, and wherein the identifying S150 one or more of the candidate locations 160_{1..j} as the potential location of the stenosis is based on the correlation values 180_{1..j}. The calculating S140 also includes:
normalizing the spatial distribution of perfused blood in the expected perfusion map 170_{1..j} and/or the actual perfusion map 150, to provide a normalized expected perfusion map, and a normalized actual perfusion map, respectively; and
wherein the correlation values 180_{1..j} are calculated using the normalized expected perfusion map and/or the normalized actual perfusion map.

The normalization takes account of inter-patient variations and consequently improves the reliability of the method. The normalization may be performed using various reference values. For instance, the normalizing may be performed based on a mean, or a mode, or a median value of the spatial distribution of perfused blood in each of the expected perfusion map 170_{1..j}, and the actual perfusion map 150, respectively. The reference value may for instance represent an image intensity in the anatomical region as in these examples, or it may be provided by a lookup table. The reference value may represent a population average in an anatomical region. An example of a normalized actual perfusion map 150 is illustrated in Fig. 3, and wherein the actual perfusion map 150 has been normalized by subtracting from the image intensity values in the actual distribution of the contrast agent, a median value for the image intensity in myocardial tissue.

In another example, a computer program product is provided. The computer program product comprises instructions which when executed by one or more processors, cause the one or more processors to carry out a method of identifying a potential location of a stenosis in a vessel. The method comprises:
receiving S110 medical image data 110 representing an anatomical region 120, the anatomical region including one or more vessels 130_{1..i} and adjoining tissue 140;
estimating S120, from the medical image data 110, an actual perfusion map 150 representing a spatial distribution of perfused blood in the adjoining tissue 140;
analyzing S130 the medical image data 110 to generate, for each of a plurality of candidate locations 160_{1..j} along the one or more vessels, an expected perfusion map 170_{1..j} representing a spatial distribution of perfused blood in the adjoining tissue 140 as a result of blood flowing downstream of the candidate location;
calculating S140, for each of a plurality of the candidate locations 160_{1..j}, i) a correlation value 180_{1..j} representing a correlation between the expected perfusion map 170_{1..j}, and the actual perfusion map 150, or ii) a perfused blood value 190_{1..j} representing an amount of perfused blood in the actual perfusion map 150 within an expected perfused blood boundary 200_{1..j} defined by a spatial extent of the perfused blood in the expected perfusion map 170_{1..j}; and
identifying S150 one or more of the candidate locations 160_{1..j} as the potential location of the stenosis based on the correlation values 180_{1..j}, or the perfused blood values 190_{1..j}, respectively.

In another example, a system 300 for identifying a potential location of a stenosis in a vessel, is provided. The system comprises one or more processors configured to:
receive S110 medical image data 110 representing an anatomical region 120, the anatomical region including one or more vessels 130_{1..i} and adjoining tissue 140;
estimate S120, from the medical image data 110, an actual perfusion map 150 representing a spatial distribution of perfused blood in the adjoining tissue 140;
analyze S130 the medical image data 110 to generate, for each of a plurality of candidate locations 160_{1..j} along the one or more vessels, an expected perfusion map 170_{1..j} representing a spatial distribution of perfused blood in the adjoining tissue 140 as a result of blood flowing downstream of the candidate location;
calculate S140, for each of a plurality of the candidate locations 160_{1..j}, i) a correlation value 180_{1..j} representing a correlation between the expected perfusion map 170_{1..j}, and the actual perfusion map 150, or ii) a perfused blood value 190_{1..j} representing an amount of perfused blood in the actual perfusion map 150 within an expected perfused blood boundary 200_{1..j} defined by a spatial extent of the perfused blood in the expected perfusion map 170_{1..j}; and
identify S150 one or more of the candidate locations 160_{1..j} as the potential location of the stenosis based on the correlation values 180_{1..j}, or the perfused blood values 190_{1..j}, respectively.

An example of the system 300 is illustrated in Fig. 2. It is noted that the system 300 may be provided with one or more additional items. For instance, the system 300 may also include one or more of: a medical imaging system 320 for providing the medical image data 110, such as for example the CT imaging system illustrated in Fig. 2; a monitor 330 for displaying the data outputted by the one or more processors 310 such as graphical representations of the vessel(s) 130_{1..i}, the candidate locations 160_{1..j}, and so forth; a patient bed 340; and a user input device (not illustrated in Fig. 2) such as a keyboard, a mouse, a touchscreen, and so forth, and which is configured to receive user input relating to operations performed by the one or more processors 310.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 300, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of identifying a potential location of a stenosis in a vessel, the method comprising:
receiving (S110) medical image data (110) representing an anatomical region (120), the anatomical region including one or more vessels (130_{1..i}) and adjoining tissue (140);
estimating (S120), from the medical image data (110), an actual perfusion map (150) representing a spatial distribution of perfused blood in the adjoining tissue (140);
analyzing (S130) the medical image data (110) to generate, for each of a plurality of candidate locations (160_{1..j}) along the one or more vessels, an expected perfusion map (170_{1..j}) representing a spatial distribution of perfused blood in the adjoining tissue (140) as a result of blood flowing downstream of the candidate location;
calculating (S140), for each of a plurality of the candidate locations (160_{1..j}), i) a correlation value (180_{1..j}) representing a correlation between the expected perfusion map (170_{1..j}), and the actual perfusion map (150), or ii) a perfused blood value (190_{1..j}) representing an amount of perfused blood in the actual perfusion map (150) within an expected perfused blood boundary (200_{1..j}) defined by a spatial extent of the perfused blood in the expected perfusion map (170_{1..j}); and
identifying (S150) one or more of the candidate locations (160_{1..j}) as the potential location of the stenosis based on the correlation values (180_{1..j}), or the perfused blood values (190_{1..j}), respectively.

2. The computer-implemented method according to claim 1, wherein the medical image data (110) represents an actual distribution of a contrast agent in the anatomical region (120), and wherein the actual perfusion map (150) is estimated based on the actual distribution of the contrast agent in the anatomical region.

3. The computer-implemented method according to claim 1 or claim 2, wherein the analyzing (S130) the medical image data (110) to generate an expected perfusion map (170_{1..j}), comprises:
identifying a path of the one or more vessels (130_{1..i}) in the anatomical region (120);
defining a plurality of candidate locations (160_{1..j}) along the path of the one or more vessels (130_{1..i}); and
generating the expected perfusion map (170_{1..j}) by modelling the spatial distribution of perfused blood in the adjoining tissue (140) as a result of blood flowing downstream of the candidate location in the absence of a stenosis in the one or more vessels (130_{1..i}).

4. The computer-implemented method according to claim 3, wherein the modelling the spatial distribution of perfused blood in the adjoining tissue (140) comprises:
assigning values to the adjoining tissue (140) downstream of the candidate location based on a distance of the tissue from the path; or
assigning a reference spatial distribution of perfused blood to the adjoining tissue (140).

5. The computer-implemented method according to claim 1 or claim 2, wherein the analyzing (S130) the medical image data (110) to generate an expected perfusion map (170_{1..j}), comprises:
identifying a path of the one or more vessels (130_{1..i}) in the anatomical region (120);
defining a plurality of candidate locations (160_{1..j}) along the path of the one or more vessels (130_{1..i}); and
generating the expected perfusion map (170_{1..j}) by modelling the spatial distribution of perfused blood in the adjoining tissue (140) as a result of blood flowing downstream of the candidate location in the presence of a candidate stenosis in the candidate location.

6. The computer-implemented method according to claim 5, wherein the generating, and the calculating (S140), are performed iteratively for a plurality of different magnitudes of the candidate stenosis, to further determine a magnitude of the stenosis at the candidate location in the vessel.

7. The computer-implemented method according to any one of claims 3-6, wherein the identifying a path of the one or more vessels (130_{1..i}) in the anatomical region (120) comprises:
segmenting the medical image data (110) to identify the path; and/or
analyzing the medical image data (110) using a neural network trained to identify the path.

8. The computer-implemented method according to any previous claim, wherein the method comprises calculating (S140), for each of a plurality of the candidate locations (160_{1..j}), i) a correlation value (180_{1..j}) representing a correlation between the expected perfusion map (170_{1..j}), and the actual perfusion map (150), and wherein the identifying (S150) one or more of the candidate locations (160_{1..j}) as the potential location of the stenosis is based on the correlation values (180_{1..j}); and
wherein the calculating (S140) further comprises:
normalizing the spatial distribution of perfused blood in the expected perfusion map (170_{1..j}) and/or the actual perfusion map (150), to provide a normalized expected perfusion map, and a normalized actual perfusion map, respectively; and
wherein the correlation values (180_{1..j}) are calculated using the normalized expected perfusion map and/or the normalized actual perfusion map.

9. The computer-implemented method according to claim 8, wherein the normalizing is performed based on a mean, or a mode, or a median value of the spatial distribution of perfused blood in each of the expected perfusion map (170_{1..j}), and the actual perfusion map (150), respectively.

10. The computer-implemented method according to any one of claims 1-7, wherein the method comprises calculating (S140), for each of a plurality of the candidate locations (160_{1..j}), ii) a perfused blood value (190_{1..j}) representing an amount of perfused blood in the actual perfusion map (150) within an expected perfused blood boundary (200_{1..j}) defined by a spatial extent of the perfused blood in the expected perfusion map (170_{1..j}), and wherein the identifying (S150) one or more of the candidate locations (160_{1..j}) as the potential location of the stenosis is based on the perfused blood values (190_{1..j}); and
wherein the method further comprises:
outputting a graphical representation of the one or more vessels (130_{1..i}); and
wherein the identifying (S150) comprises:
a) indicating the perfused blood values (190_{1..j}) in the graphical representation at the corresponding candidate locations (160_{1..j}); and/or
b) calculating a gradient of the perfused blood values (190_{1..j}) along the vessel, and identifying one or more of the candidate locations (160_{1..j}) as the potential location of the stenosis based on a magnitude of the gradient of the perfused blood values (190_{1..j}).

11. The computer-implemented method according to claim 1 or claim 10, wherein the method comprises calculating (S140), for each of a plurality of the candidate locations (160_{1..j}), ii) a perfused blood value (190_{1..j}) representing an amount of perfused blood in the actual perfusion map (150) within an expected perfused blood boundary (200_{1..j}) defined by a spatial extent of the perfused blood in the expected perfusion map (170_{1..j}), and wherein the identifying (S150) one or more of the candidate locations (160_{1..j}) as the potential location of the stenosis is based on the perfused blood values (190_{1..j}); and
wherein the calculating (S140) comprises, for each of the candidate locations (160_{1..j}):
determining the expected perfused blood boundary (200_{1..j}) based on a spatial extent of the perfused blood in the expected perfusion map (170_{1..j});
mapping the expected perfused blood boundary (200_{1..j}) onto the actual perfusion map (150); and
analyzing the spatial distribution of perfused blood in the actual perfusion map (150) within the expected perfused blood boundary (200_{1..j}), to provide the perfused blood value (190_{1..j});
or
mapping the expected perfusion map (170_{1..j}) onto the actual perfusion map (150) to define the expected perfused blood boundary (200_{1..j}) in the actual perfusion map;
weighting the spatial distribution of perfused blood in the actual perfusion map (150) within the expected perfused blood boundary (200_{1..j}), with the spatial distribution of perfused blood in the expected perfusion map (170_{1..j}); and
analyzing the weighted spatial distribution to provide the perfused blood value (190_{1..j}).

12. The computer-implemented method according to any previous claim, wherein the method further comprises segmenting the medical image data (110) to identify the adjoining tissue (140); and
wherein the estimating (S120), and the analyzing (S130) the medical image data (110), are performed using the segmented medical image data.

13. The computer-implemented method according to any previous claim, wherein the identifying (S150) one or more of the candidate locations (160_{1..j}) as the potential location of the stenosis, comprises:
outputting a graphical representation of the anatomical region (120) including the one or more vessels (130_{1..i}) and the adjoining tissue (140); and
displaying, in the graphical representation, one or more markers indicating the one or more candidate locations (160_{1..j}) having the highest correlation values (180_{1..j}), or the one or more candidate locations (160_{1..j}) corresponding to a local maximum in a magnitude of a gradient of the perfused blood values (190_{1..j});
and/or
adapting the graphical representation of the one or more vessels (130_{1..i}) to indicate the identified potential location of the stenosis;
and/or
displaying in the graphical representation, a ranked list comprising the one or more candidate locations (160_{1..j}) identified as the potential location of the stenosis.

14. The computer-implemented method according to claim 13, wherein the method further comprises overlaying the graphical representation of the anatomical region (120) with the actual perfusion map (150).

15. The computer-implemented method according to any previous claim, wherein the identifying (S150) one or more of the candidate locations (160_{1..j}) as the potential location of the stenosis, respectively comprises:
i) identifying as the potential location of the stenosis, one or more of the candidate locations (160_{1..j}) having the highest correlation values (180_{1..j}); or
ii) calculating a gradient of the perfused blood values (190_{1..j}) along the vessel, and identifying as the potential location of the stenosis, one or more candidate locations (160_{1..j}) corresponding to a local maximum in a magnitude of the gradient of the perfused blood values (190_{1..j}).
